# EUROPEAN PATENT APPLICATION

(11) **EP 4 631 501 A1**
(43) Date of publication of application: **15.10.2025**
(21) Application number: 24169194.8
(22) Date of filing: 09.04.2024
(51) Int. Cl.: A61K 31/35, A61K 31/4545, A61K 31/513, A61K 31/519, A61K 31/55, A61K 31/675, A61K 31/704, A61K 31/7048, A61K 39/00, A61K 45/06, A61N 5/00, C07K 16/00, A61P 35/00

(54) **COMBINATIONS OF A CALCIUM HOMEOSTASIS DISRUPTOR WITH A DEATH RECEPTOR 5 AGONIST FOR TREATING CANCER**

(71) Applicant: Stichting Het Nederlands Kanker Instituut- Antoni van Leeuwenhoek Ziekenhuis, 1066 CX Amsterdam (NL)
(72) Inventor: Bernards, Rene, 1066 CX Amsterdam (NL); Wang, Liqin, 1066 CX Amsterdam (NL); Casagrande Raffi, Gabriele, 1066 CX Amsterdam (NL)
(74) Representative: Pot, Emil

(57) **Abstract**

The invention relates to a calcium homeostasis disruptor in combination with a selective Death Receptor 5 (DR5) agonist for use in a method of treating a patient suffering from a tumor. The invention further relates to a pharmaceutical composition, comprising a calcium homeostasis disruptor in combination with a selective Death Receptor 5 (DR5) agonist and, optionally, an inducer of senescence. The invention further relates to methods of treating a patient having a tumor, and to a calcium homeostasis disruptor in combination with a selective DR5 agonist, for use in a method of selectively killing senescent cells, including senescent cancer cells.

## Description

### Field of the invention

The invention pertains to the field of cancer treatment, in particular to the field of synergistic combinations of senolytic agents.

### Background

A variety of stresses can trigger senescence, and it is considered a fail-safe mechanism against oncogenic transformation. Furthermore, senescence has been recognized as a stress response to anti-cancer therapies. Though at first glance, such a proliferation arrest may seem advantageous, multiple studies have highlighted that the SASP produced by senescent tumor cells represents a potentially double-edged sword for tumor control. On the one hand, the SASP can suppress tumor growth by inducing an immunological response. On the other hand, prolonged exposure to SASP can potentially be harmful, causing chronic inflammation, immunosuppression, stimulating epithelial-to-mesenchymal transition, inducing a stem cell-like state, or promoting tumor migration and metastasis. Therefore, while the senescence growth arrest may represent an initial desirable outcome of treatment, prolonged presence of senescent cells can be deleterious.

Senolytics have emerged as a promising therapeutic strategy to selectively eliminate senescent cells that accumulate in tissues during aging and in age-related diseases, including cancer. The elimination of these cells is believed to improve tissue function and delay or even reverse the aging process. In cancer therapy, senolytics could be combined with pro-senescence therapies to prevent the harmful effects of persistent senescent cells in tumors and during cancer treatment. However, despite the potential benefits of this approach, the development of senolytic drugs has been challenging due to the lack of universal senolytic agents that can selectively target senescent cancer cells in a context-independent manner with an acceptable toxicity profile.

### Summary of the invention

The invention is summarized in the following embodiments:
- Embodiment 1: A calcium homeostasis disruptor in combination with a selective Death Receptor 5 (DR5) agonist for use in a method of treating a patient suffering from a tumor.
- Embodiment 2: The calcium homeostasis disruptor for use according to embodiment 1, wherein the disruptor interacts with the mitochondrial inner membrane protein SLC25A23.
- Embodiment 3: The calcium homeostasis disruptor for use according to embodiments 1 and 2, wherein the disruptor is the ionophore antibiotic salinomycin.
- Embodiment 4: The calcium homeostasis disruptor for use according to any one of embodiments 1 - 3, wherein the patient has been exposed to a treatment that induces senescence in tumor cells prior to the treatment according to any one of embodiments 1 - 3.
- Embodiment 5: The calcium homeostasis disruptor for use according to embodiment 4, wherein the senescence inducer comprises at least one of palbociclib, alisertib, TAS-119, PF-06873600, CFI-400945, etoposide, doxorubicin, 5-FU and barasertib, [other chemotherapeutic agents], gamma-radiation.
- Embodiment 6: The calcium homeostasis disruptor for use according to embodiment 4, wherein the inducer of senescence comprises at least one of chemotherapy, ionizing radiation, a CDK4/6 inhibitor, a polo-like kinase 4 (PLK4) inhibitor, a topoisomerase II inhibitor, an aurora kinase B inhibitor.
- Embodiment 7: The calcium homeostasis disruptor for use according any one of embodiments 4-6, wherein the inducer of senescence, the calcium homeostasis disruptor and the selective Death Receptor 5 (DR5) agonist are provided sequentially to the patient.
- Embodiment 8: The calcium homeostasis disruptor for use according any one of embodiments 1 - 7, wherein the tumor is a solid tumor such as lung cancer, breast cancer, colorectal cancer and/or liver cancer.
- Embodiment 9: The selective DR5 agonist for use according any one of embodiments 1 - 7, wherein the selective DR5 agonist is an antibody, preferably a human or humanized antibody, more preferably a human or humanized IgA or IgA-like antibody.
- Embodiment 10: A pharmaceutical composition, comprising an inducer of senescence a calcium homeostasis disruptor and a selective Death Receptor 5 (DR5) agonist.
- Embodiment 11: The pharmaceutical preparation according to claim 10, for use in a method of treating a patient suffering from a tumor.
- Embodiment 12: A method of treating a patient having a tumor with a combination of an inducer of senescence, a calcium homeostasis disruptor and a selective DR5 agonist, comprising administering an inducer of senescence to said patient, followed by administering a calcium homeostasis disruptor followed by administering a selective DR5 agonist having an in vivo half-life of less than 20 days, optionally in combination with a BRD2 inhibitor.
- Embodiment 13: The method of embodiment 12, wherein the calcium homeostasis disruptor is provided at least 24 hours following the inducer of senescence.
- Embodiment 14: The method of embodiment 12, wherein the DR5 agonist is provided at least 48 hours following the inducer of senescence.
- Embodiment 15: The method according to any one of embodiments 12-14, wherein the tumor is a solid tumor such as lung cancer, breast cancer, colorectal cancer and/or liver cancer.
- Embodiment 16: A method of treating a patient having a pathology involving senescent cells with a calcium homeostasis disruptor followed by a selective DR5 agonist.

### Definitions

Various terms relating to the methods, compositions, formulations, uses and other aspects of the present invention are used throughout the specification and claims. Such terms are to be given their ordinary meaning in the art to which the invention pertains, unless otherwise indicated. Other specifically defined terms are to be construed in a manner consistent with the definition provided herein. Although any methods and materials similar or equivalent to those described herein can be used in the practice for testing of the present invention, the preferred materials and methods are described herein.

Methods of carrying out the conventional techniques used in methods of the invention will be evident to the skilled worker. The practice of conventional techniques in molecular biology, biochemistry, computational chemistry, cell culture, recombinant DNA, bioinformatics, genomics, sequencing and related fields are well-known to those of skill in the art and are discussed, for example, in the following literature references: Sambrook et al., Molecular Cloning. A Laboratory Manual, 2nd Edition, Cold Spring Harbor Laboratory Press, Cold Spring Harbor, N. Y., 1989; Ausubel et al., Current Protocols in Molecular Biology, John Wiley & Sons, New York, 1987 and periodic updates; and the series Methods in Enzymology, Academic Press, San Diego.

"A," "an," and "the": these singular form terms include plural referents unless the content clearly dictates otherwise. The indefinite article "a" or "an" thus usually means "at least one". Thus, for example, reference to "a cell" includes a combination of two or more cells, and the like.

"About" and "approximately": these terms, when referring to a measurable value such as an amount, a temporal duration, and the like, is meant to encompass variations of ±20% or ±10%, more preferably ±5%, even more preferably ±1%, and still more preferably ±0.1% from the specified value, as such variations are appropriate to perform the disclosed methods. Additionally, amounts, ratios, and other numerical values are sometimes presented herein in a range format. It is to be understood that such range format is used for convenience and brevity and should be understood flexibly to include numerical values explicitly specified as limits of a range, but also to include all individual numerical values or sub-ranges encompassed within that range as if each numerical value and sub-range is explicitly specified. For example, a ratio in the range of about 1 to about 200 should be understood to include the explicitly recited limits of about 1 and about 200, but also to include individual ratios such as about 2, about 3, and about 4, and sub-ranges such as about 10 to about 50, about 20 to about 100, and so forth.

"And/or": The term "and/or" refers to a situation wherein one or more of the stated cases may occur, alone or in combination with at least one of the stated cases, up to with all of the stated cases.

"Comprising": this term is construed as being inclusive and open ended, and not exclusive. Specifically, the term and variations thereof mean the specified features, steps or components are included. These terms are not to be interpreted to exclude the presence of other features, steps or components.

Exemplary": this terms means "serving as an example, instance, or illustration," and should not be construed as excluding other configurations disclosed herein.

"Senescence", as is used herein, refers to a state of a cell that is characterized by having an essentially permanent growth arrest in the G1 or G2/M phase of the cell cycle. A senescent cell is essentially irresponsive to proliferation-cues. The term "senescent cell", as used herein, includes a cell that is characterized by (1) an essentially permanent growth arrest; (2) loss of proliferation markers such as cyclin A, MCM-3 and/or PCNA; (3) insensitivity to growth cues; (4) induction of a senescence-associated β-gaiactosidase (SA-B-Gal); and (5) nuclear export of alarmin, a High Mobility Group Box 1 protein. The phenomenon of senescence can occur at the end of the proliferative lifespan of normal cells or in normal or tumor cells in response to, for example, chemotherapeutic agents, radiation, or other cellular insults. Senescent cells often remain metabolically active and commonly adopt an immunogenic phenotype consisting of a pro-inflammatory secretome. A senescent cell often has upregulated expression of a cell cycle inhibitor like p16/p21.

"Pathology involving senescent cells", as is used herein, refers to pathologies such as osteoporosis, frailty, cardiovascular diseases, osteoarthritis, pulmonary fibrosis, renal diseases, neurodegenerative diseases, hepatic steatosis, metabolic dysfunction, and senescent fibroblast-mediated pathologies such as idiopathic pulmonary fibrosis. A pathology involving senescent cells may also be referred to as an age-related disease.

"Inducer of senescence", as is used herein, refers to the induction of a cellular stress response that results in an essentially permanent growth arrest of the cell. Senescence can be triggered by a diverse set of signals, including shortening of telomeres, DNA damage, activation of oncogenes, and oxidative stress. In the context of this invention, an inducer or senescence is preferably selected from a chemotherapeutic agent, ionizing radiation, a CDK4/6 inhibitor, a Polo-like kinase 4 (PLK4) inhibitor, a Topoisomerase II inhibitor, and an Aurora kinase inhibitor, preferably an Aurora kinase B inhibitor.

"Cyclin-dependent kinase 4/6 (CDK4/6)", as is used herein, refers to two closely related members of a family of serine/threonine protein kinases that participate in cell cycle regulation, CDK4 and CDK6. Both members are cyclin D-dependent kinases that regulate entry into the DNA synthetic (S) phase of the cell-division cycle in a retinoblastoma protein-dependent manner.

"Inhibitor of cyclin-dependent kinase 4/6", as is used herein, refers to a molecule that inhibits CDK4/6. A preferred CDK4/6 inhibitor is selective for CDK4/6, when compared to other serine/threonine protein kinases such as CDK1 and CDK2, meaning that the molecule is at least two times more potent, preferably at least five times more potent, in inhibiting CDK4/6, when compared to other serine/threonine protein kinases.

"Polo-like kinase 4 (PLK4)", as is used herein, refers to a serine/threonine protein kinase that plays a central role in centriole duplication. The human gene encoding PLK4 resides on chromosome 4q28.1, and is characterized by HGNC entry code 11397; Entrez Gene entry code 10733; and Ensembl entry code ENSG00000142731. The PLK4 protein is characterized by UniProt entry code O00444.

"PLK4 inhibitor", as is used herein, refers to a molecule that inhibits PLK4. A preferred PLK4 inhibitor is selective for PLK4, when compared to other polo-like serine/threonine protein kinases such as PLK1, PLK2 and PLK3, meaning that the molecule is at least two times more potent, preferably at least five times more potent, in inhibiting PLK4, when compared to other serine/threonine protein kinases such as other polo-like serine/threonine protein kinases.

"Topoisomerase II", as is used herein, refers to a DNA Type IIA topoisomerase that is involved in the separation of chromosomal daughter strands during replication. Failure to separate these strands leads to cell death. The human gene encoding topoisomerase II resides on chromosome 17q21.2, and is characterized by HGNC entry code 11989; Entrez Gene entry code 7153; and Ensembl entry code ENSG00000131747. The topoisomerase II protein is characterized by UniProt entry code P11388.

"Topoisomerase II inhibitor", as is used herein, refers to a molecule that inhibits topoisomerase II. A preferred topoisomerase II inhibitor is selective for topoisomerase II, when compared to other topoisomerases such as topoisomerase I and topoisomerase III, meaning that the molecule is at least two times more potent, preferably at least five times more potent, in inhibiting topoisomerase II, when compared to other topoisomerases such as topoisomerase I and topoisomerase III.

"Aurora kinase B", as is used herein, refers to serine/threonine protein kinase that is a component of the chromosomal passenger complex that acts as a key regulator of mitosis. The human gene encoding aurora kinase B resides on chromosome 17p13.1, and is characterized by HGNC entry code 11390; Entrez Gene entry code 9212; and Ensembl entry code ENSG00000178999. The aurora kinase B protein is characterized by UniProt entry Q96GD4.

"Aurora kinase B inhibitor", as is used herein, refers to a molecule that inhibits aurora kinase B. A preferred aurora kinase B inhibitor is selective for aurora kinase B, when compared to other aurora kinases such as aurora kinase A and aurora kinase C, meaning that the molecule is at least two times more potent, preferably at least five times more potent, in inhibiting aurora kinase B, when compared to other aurora kinases such as aurora kinase A and aurora kinase C.

"Death Receptor 5 or DR5", as is used herein, refers to protein member 10b of the tumor necrosis factor (TNF) Receptor Superfamily. Alternative names are TRAILR2 and TRICK2. The human gene encoding DR5 resides on chromosome 8p21.3, and is characterized by HGNC entry code 11905; Entrez Gene entry code 8795; and Ensembl entry code ENSG00000120889. The DR5 protein is characterized by UniProt entry code 014763.

"DR5 agonist", as is used herein, refers to a molecule such as an antibody that binds and activates DR5. DR5 harbors a death domain, a stretch of about 90 amino acid residues that is required and sufficient to activate the apoptotic machinery. Binding and activation of DR5 by a DR5 agonist thus results in the induction of apoptosis.

"Selective DR5 agonist", as is used herein, refers to a molecule that binds and activates specifically DR5. Binding of a selective DR5 agonist to DR5 is at least two times more potent, preferably at least five times more potent, in activating DR5, when compared to other death receptor proteins such as DR4.

"Peptide", as used herein, refers to a molecule with an amino acid chain of between 5 and 100 amino acid residues, preferably between 10 and 50 amino acid residues. The term peptide includes a peptide in which one or more of the amino acid monomers have been modified, for example by acetylation, amidation and/or glycosylation.

"Peptide analogue", as used herein, refers to peptidomimetics which are or which comprise small peptide-like chains such as peptoids and β-peptides designed to mimic a peptide. The altered chemical structure is preferably designed to adjust one or more properties such as, for example, stability, of a peptide.

"Combination", as is used herein, refers to the administration of effective amounts of an inducer of senescence and a combination of Salinomycin or other calcium homeostasis inhibitor and a selective DR5 agonist to a patient in need thereof. Said inducer of senescence and a combination of Salinomycin or other calcium homeostasis inhibitor and a selective DR5 agonist may be provided in one pharmaceutical preparation, or as two distinct pharmaceutical preparations.

"Antibody", as is used herein, includes reference to classical heterodimers of heavy and light chain antibodies, single heavy chain variable domain antibody such as a camelid VHH, a shark immunoglobulin-derived variable new antigen receptor, and scFv, tandem scFv, scFab, and improved scFab (Koerber et al., 2015. J Mol Biol 427: 576-86). The heavy and light chains of classical antibodies comprise a variable region (V region) and a constant or C region. As described herein, the amino acid sequence and structure of the variable region of heavy and light chains of classical antibodies is comprised of four framework regions or `FR', which are referred to herein as 'Framework region 1' or 'FRI'; as 'Framework region 2' or'FR2'; as 'Framework region 3' or `FR3'; and as 'Framework region 4' or `FR4', respectively; which framework regions are interrupted by three complementary determining regions or `CDR' s', which are referred to herein as 'Complementarity Determining Region I' or 'CDR1'; as 'Complementarity Determining Region 2' or 'CDR2'; and as 'Complementarity Determining Region 3' or 'CDR3', respectively. The term antibody also includes an antibody-like molecule that is not structurally related to an antibody. Such antibody-like molecules include, for example, a designed ankyrin repeat protein, a binding protein that is based on a Z domain of protein A, a binding protein that is based on a fibronectin type III domain, engineered lipocalin, and a binding protein that is based on a human Fyn SH3 domain (Skerra, 2007. Current Opinion Biotechnol 18: 295-304; Skrlec et al., 2015. Trends Biotechnol 33: 408-418).

"Anti-DR5 IgA or IgA-like antibody", as is used herein, refers to any and all anti-DR5 antibodies that comprise at least part of a CD89-interacting domain in the Cα3 domain, including at least amino acid residues L441A442 (Pleass et al., 1999. JBC 274: 23508-23514). The inclusion of an IgA-derived CD89-interacting domain with at least amino acid residues L441A442 will contribute to a shortened in vivo half-life of the anti-DR5 IgA or IgA-like antibody of less than 20 days, such as 3-9 days.

"Selective binding", or grammatical variations thereof, as used herein, refers to the number of different types of antigens or their epitopes to which a particular antibody can bind. The specificity of an antibody can be determined based on affinity. A specific antibody preferably has a binding affinity Kd for its epitope of less than 10-7 M, preferably less than 10-8 M, most preferable less than 10-9 M.

"Format", or "antibody format', as is used herein, refers to the class or isotype of an antibody, which for human antibodies is selected from immunoglobulins (Ig) IgA, IgD, IgE, IgG, or IgM, which are in part determined by the constant region. The constant region includes sites involved in interactions with other components of the immune system.

The terms "reduced" or "eliminated" or "disruption" or "disrupted" are used interchangeably herein to refer to any genetic modification that decreases or eliminates expression and/or the functional activity of the nucleic acid or an expression product thereof. For example, disruption of a gene includes within its scope any genetic modification that decreases or eliminates expression of the gene and/or the functional activity of a corresponding gene product ( e.g ., mRNA and/or protein). Genetic modifications include complete or partial inactivation, suppression, deletion, interruption, blockage, or down- regulation of a nucleic acid (e.g., a gene). Illustrative genetic modifications include, but are not limited to, gene knockout, inactivation, mutation (e.g., insertion, deletion, point, or frameshift mutations that disrupt the expression or activity of the gene product), or use of inhibitory nucleic acids (e.g., inhibitory RNAs such as sense or antisense RNAs, molecules that mediate RNA interference such as siRNA, shRNA, miRNA; etc.), inhibitory polypeptides (e.g., antibodies, polypeptide-binding partners, dominant negative polypeptides, enzymes etc.) or any other molecule that inhibits the activity of the LCK gene or level or functional activity of an expression product of the LCK gene.

By "pharmaceutically acceptable carrier" is meant a solid or liquid filler, diluent or encapsulating substance that can be safely used in topical or systemic administration to an animal, preferably a mammal, including humans. Representative pharmaceutically acceptable carriers include any and all solvents, dispersion media, coatings, surfactants, antioxidants, preservatives (e.g., antibacterial agents, antifungal agents), isotonic agents, absorption delaying agents, salts, preservatives, drugs, drug stabilizers, gels, binders, excipients, disintegration agents, lubricants, sweetening agents, flavoring agents, dyes, such like materials and combinations thereof, as would be known to one of ordinary skill in the art (see, for example, Remington's Pharmaceutical Sciences, 18th Ed. Mack Printing Company, 1990, pp. 1289-1329, incorporated herein by reference). Except insofar as any conventional carrier is incompatible with the active ingredient(s), its use in the pharmaceutical compositions is contemplated.

The terms "cancer" and "cancerous" refer to or describe the physiological condition in mammals that is typically characterized in part by unregulated cell growth. As used herein, the term "cancer" refers to non-metastatic and metastatic cancers, including early stage and late stage cancers. The term "precancerous" refers to a condition or a growth that typically precedes or develops into a cancer. By "non-metastatic" is meant a cancer that is benign or that remains at the primary site and has not penetrated into the lymphatic or blood vessel system or to tissues other than the primary site. Generally, a non-metastatic cancer is any cancer that is a Stage 0, 1, or II cancer, and occasionally a Stage III cancer. By "early stage cancer" is meant a cancer that is not invasive or metastatic or is classified as a Stage 0, I, or II cancer. The term "late stage cancer" generally refers to a Stage III or Stage IV cancer, but can also refer to a Stage II cancer or a sub-stage of a Stage II cancer. One skilled in the art will appreciate that the classification of a Stage II cancer as either an early stage cancer or a late stage cancer depends on the particular type of cancer.

The term "cancer" includes but is not limited to, breast cancer, large intestinal cancer, lung cancer, small cell lung cancer, gastric (stomach) cancer, liver cancer, blood cancer, bone cancer, pancreatic cancer, skin cancer, head or neck cancer, cutaneous or intraocular melanoma, uterine sarcoma, ovarian cancer, rectal or colorectal cancer, anal cancer, colon cancer, fallopian tube carcinoma, endometrial carcinoma, cervical cancer, vulval cancer, squamous cell carcinoma, vaginal carcinoma, Hodgkin's disease, non-Hodgkin's lymphoma, esophageal cancer, small intestine cancer, endocrine cancer, thyroid cancer, parathyroid cancer, adrenal cancer, soft tissue tumor, urethral cancer, penile cancer, prostate cancer, chronic or acute leukemia, lymphocytic lymphoma, bladder cancer, kidney cancer, ureter cancer, renal cell carcinoma, renal pelvic carcinoma, CNS tumor, glioma, astrocytoma, glioblastoma multiforme, primary CNS lymphoma, bone marrow tumor, brain stem nerve gliomas, pituitary adenoma, uveal melanoma (also known as intraocular melanoma), testicular cancer, oral cancer, pharyngeal cancer or a combination thereof. Cancers that may be treated include tumors that are not vascularized, or not yet substantially vascularized, as well as vascularized tumors. The cancers may comprise non-solid tumors (such as hematological tumors, for example, leukemias and lymphomas) or may comprise solid tumors. Types of cancers to be treated with the CARs of the invention include, but are not limited to, carcinoma, blastoma, and sarcoma, and certain leukemia or lymphoid malignancies, benign and malignant tumors, and malignancies e.g., sarcomas, carcinomas, and melanomas. Adult tumors/cancers and pediatric tumors/cancers are also included.

The term "administering" refers to contacting, applying, injecting, transfusing or providing a composition of the present invention to a subject.

The term "treating" as used herein may refer to (1) preventing or delaying the appearance of one or more symptoms of the disorder; (2) inhibiting the development of the disorder or one or more symptoms of the disorder; (3) relieving the disorder, i.e., causing regression of the disorder or at least one or more symptoms of the disorder; and/or (4) causing a decrease in the severity of one or more symptoms of the disorder.

The term "subject" as used throughout the specification is to be understood to mean a human or may be a domestic or companion animal. While it is particularly contemplated that the methods of the invention are for treatment of humans, they are also applicable to veterinary treatments, including treatment of companion animals such as dogs and cats, and domestic animals such as horses, cattle and sheep, or zoo animals such as primates, felids, canids, bovids, and ungulates. The "subject" may include a person, a patient or individual, and may be of any age or gender.

### Detailed description

To address the lack of universal senolytic agents that can selectively target senescent cancer cells in a context-independent manner with an acceptable toxicity profile, we have used unbiased genetic screens to identify vulnerabilities in senescent cancer cells that can be targeted by novel senolytic agents. These screens involve the systematic loss-of-function analysis of genes that are essential for the survival of senescent cells but not normal cells.

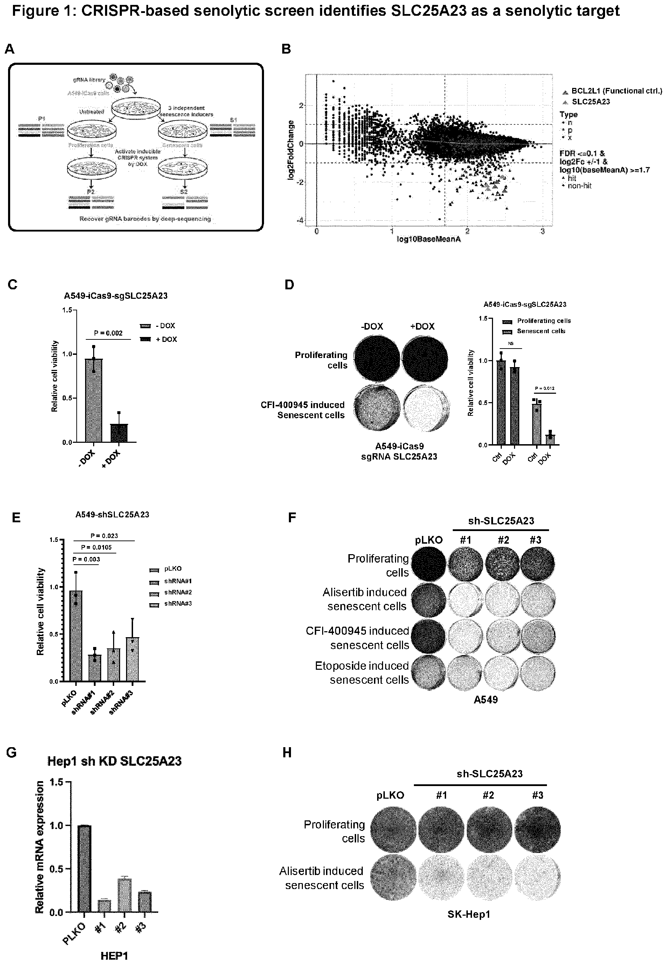

To identify genes whose inactivation causes cell death selectively in senescent cancer cells, we developed a genome-scale loss of function genetic screen using a doxycycline-inducible CRISPR/Cas9 vector system (Fig. 1A). We used as the screening model A549 KRAS mutant lung cancer cells treated with an aurora kinase A inhibitor alisertib to induce senescence. We first introduced the lentiviral gRNA library in proliferating A549 cells, followed by selection for lentiviral integration. The gRNA-expressing cells were treated with alisertib to induce senescence. After this, CAS9 expression was activated by doxycycline treatment to induce gene knock-outs. The senescent cell populations were cultured for 10 days to allow for the depletion of gRNAs in the senescent cells (Fig. 1A). A control arm of proliferating cells was also included in the screen. We selected sgRNAs preferentially depleted from cells made senescent by alisertib, with little or no effect in the proliferating arms. Using this criterium, we surprisingly identified SLC25A23, a mitochondrial inner membrane protein that controls calcium homeostasis that affects mitochondrial complex I assembly and respiratory chain function, as the top hit (Fig. 1B). For validation, we used a gRNAs targeting SLC25A23 in A549 embedded doxycycline-inducible Cas9 (A549-iCas9) cell. The gRNA can reduce the gene expression in the mRNA level of SLC25A23 (Fig. 1C). Colony formation assays confirmed that depletion of SCL25A23 can selectively kill the senescent cells induced by alisertib but not the proliferating cells (Figure 1D). To confirm this phenotype, we also used three independent shRNAs targeting SLC25A23 in A549 cells. The results showed that the shRNAs could effectively suppress the expression of SLC25A23. As a consequence, the SLC25A23 suppressed cells became highly sensitive to various senescence inducers, not only alisertib but also the PLK4 inhibitor CFI-400945 and the topoisomerase II inhibitor etoposide (Fig. 1E-F), this phenotype can be also confirmed in a liver cancer model SK-Hep1(Fig. 1G-H).

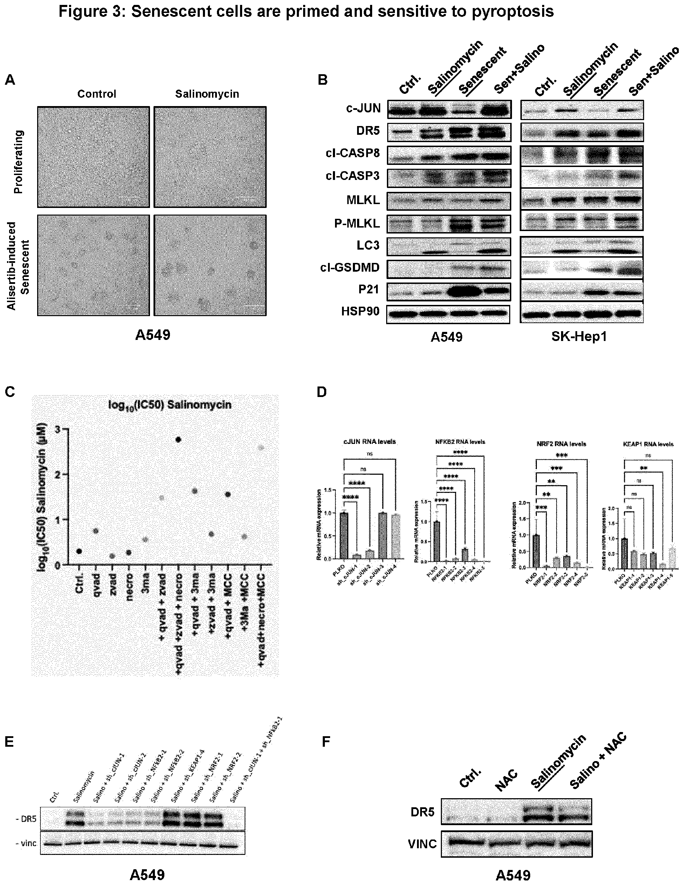

The function of SLC25A23 is to control mitochondrial calcium homeostasis, which may be critical for the survival of senescent cells. We tested an ionophore antibiotic, salinomycin, to affect calcium homeostasis in the cells. These experiments showed surprisingly that salinomycin indeed resulted in the preferential killing of senescent cells over proliferating cells in multiple senescent cell models, including lung (A549), breast (MDA-MB-231, MCF-7, T47D) and liver (SK-Hep1) cancer models, which are treated with different senescent inducers (Fig. 3A-B).

Under the microscope, we observed that treating senescent cells with salinomycin caused a PANoptosis phenotype, a mixture of distinct cell death including, apoptosis, necroptosis, pyroptosis and autophagy. (Fig. 3A). This notion was also supported by the western blot analysis. We observed that an upregulation of death receptor 5 (DR5), cleavage of caspase 3 and 8 (cl-CASP8 and 3), indicates extrinsic apoptosis activation. We also observed increased phosphorylation of mixed lineage kinase domain-like protein (MLKL), upregulation of Microtubule-associated protein 1A/1B-light chain 3 (LC3) and cleaved Gasdermin-D (cl-GSDMD), demonstrates the activation of necrosis, hyper-autophagy and pyroptosis (Figure 3B). This PANoptosis phenotype is also confirmed by the functional rescue experiment with the inhibitors of autophage (3MA), necroptosis (Necrostatin-1), apoptosis (Z-VAD-FMK), pyroptosis (MCC950) or Q-VD-Oph, a pan-caspases inhibitor, which inactivates Caspase 1, 3, 7, 8, 9. Our data showed that only the combination of death inhibition were capable to rescue salinomycin-mediated senolysis, but solo-inhibition of each cell death failed to do so (Fig. 3C). In Figure 3B, we noticed the upregulation of cJUn and DR5 by salinomycin treatment. This has been denoted by the past studies showing salinomycin could modulate autophagy to induce ROS (Verdoodt 2012). The elevated ROS led to cJun upregulation and NF-kB activation to increase the DR5 expression (Fu 2010). To validated molecular mechanism in our senescent models, we knocked-down cJun, NF-kB2 and Nrf2 using shRNA. The results showed that the suppression of cJun or NF-kB2 prevented DR5 upregulation. As the opposite, the suppression of Nrf2, a master transcription factor of anti-oxidation genes, led to a massive DR5 induction (Fig. 3D-E). Next, we also confirmed that a ROS scavenger N-Acetyl Cysteine (NAC) could reserve the DR5 upregulation in salinomycin treated cells (Fig. 3F). This further supported that the DR5 induction mechanism was through ROS.

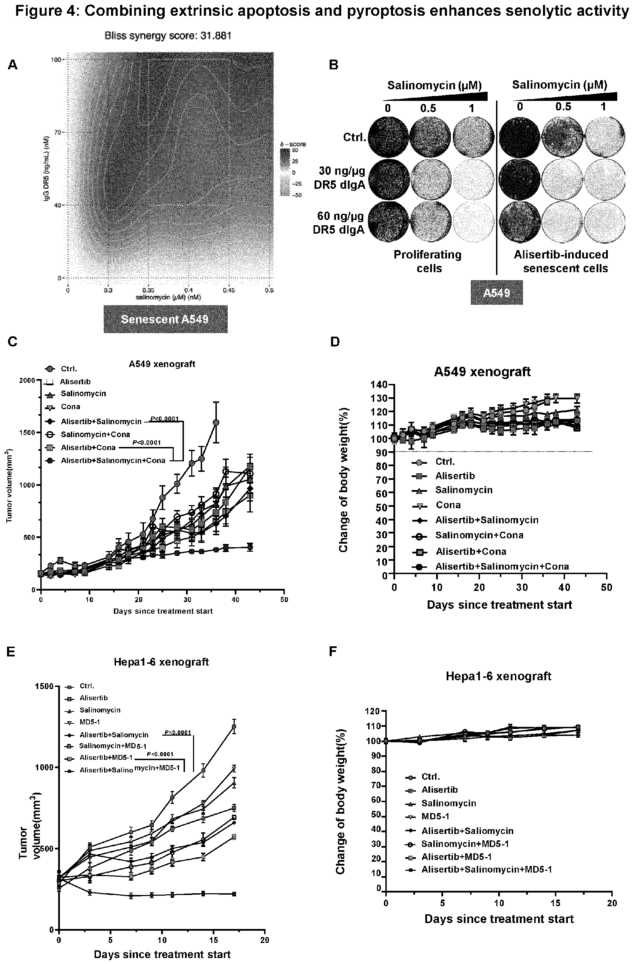

In order to obtain a synergistic effect, we combed low doses of DR5 agonist antibody either IgG-conatumumab or IgA-conatumumab with salinomycin, as senolytic cocktails. The combination result demonstrated that the cocktails have a surprisingly synergistically senolytic effect in the alisertib induced senescent A549 model (Fig. 4A-B). To test this senolytic cocktail in vivo, we used both immunodeficient and immunocompetent mouse models. We engrafted A549 cells into immunodeficient nude mice and Hepa1-6 liver cancer cells in syngeneic immunocompetent C57BL/6 mice. After tumors were established, we treated with alisertib to induce senescence and combinations of the two senolytic drugs. Treatment with single-agent alisertib, salinomycin or DR5 agonist antibodies (conatumumab for A549 and mouse DR5 antibody MD5-1 for Hepa1-6) resulted in limited tumor growth inhibition. Treatment with the combination of two drugs resulted in some tumor control, but the strongest effect was seen with the three-way combination, without affecting bodyweight of the mice (Fig. 5C-F). The same result was confirmed using 5FU and doxorubicin as senescent inducers (Fig. 5G-5L).

Together with the data above, we discovered that combining salinomycin and DR5 agonist antibodies is an efficient senolytic cocktail to eliminate therapy-induced senescent cells, as can be used for a novel cancer treatment.

### Figure legends

**Figure 1****: CRISPR-based senolytic screen identifies SLC25A23 as a senolytic target**
   (A). Schematic of iCas9-associated senolytic CRISPR screen workflow. A549 cells were infected with a doxycycline-inducible Cas9 (iCas9) lentiviral vector. Next, Brunello lentiviral whole genome-wide gRNA collection virus was introduced to the A549-iCas9 cells. These infected cells were firstly treated with 0.5 µ M alisertib for 7 days to drive them into senescence. Afterwards, these cells were suspended from alisertib then switched to 1 µg/ml doxycycline (DOX) treatment for 10 days. Non-senescent cells were included as the control arm to filter out the straight lethal genes and also treated with doxycycline for. Changes in library representation after 10 days doxycycline treatment were determined by Illumina deep-sequencing.
   (B). gRNAs prioritized for further analysis were selected by the fold depletion of abundance in in S2 and S1 samples compared with that in P2 and P2 samples, using methods as described previously (Evers et al., Nat Biotech, 2016). SLC25A23 was identified as the top hit. BCL2L1 were considered as a functionally positive control.
      I. Real-time PCR analysis of SLC25A23 in A549-iCas9 cells stably infected with a gRNA-SLC25A23 lentiviral vector. mRNA was harvested after 4 days 1 µg/ml DOX treatment. GAPDH served as loading control.
   (D). Cell viability was assessed by colony formation assay in the A549 cells stably infected with doxycycline-inducible Cas9 (iCas9) lentiviral vectors and a guide RNAs target SLC25A23 lentiviral vector. The cells were firstly treated with 0.5 µM alisertib for 7 days to induce senescence. Afterwards, the senescent cells were suspended from alisertib then re-seeded in a 6-well plate and switched to 1 µg/ml DOX treatment for 10 days. Proliferating cells were taken as a control during doxycycline treatment.
   (E). Real-time PCR analysis of SLC25A23 in A549 cells stably infected with three independent shRNA-SLC25A23 lentiviral vectors. mRNA was harvested after 3 days post-puromycin selection. GAPDH served as loading control.
   (F). Cell viability was assessed by colony formation assay in the A549 cells stably infected with with three independent shRNA-SLC25A23 lentiviral vectors. The cells were treated with 0.5 µM alisertib for 7 days. pLKO empty vectors were take a control.
   (G). Real-time PCR analysis of SLC25A23 in SK-Hep1 cells stably infected with three independent shRNA-SLC25A23 lentiviral vectors. mRNA was harvested after 3 days post-puromycin selection. GAPDH served as loading control.
   (H). Cell viability was assessed by colony formation assay in the SK-Hep1 cells stably infected with with three independent shRNA-SLC25A23 lentiviral vectors. The cells were treated with 0.5 µM alisertib for 7 days. pLKO empty vectors were take a control.

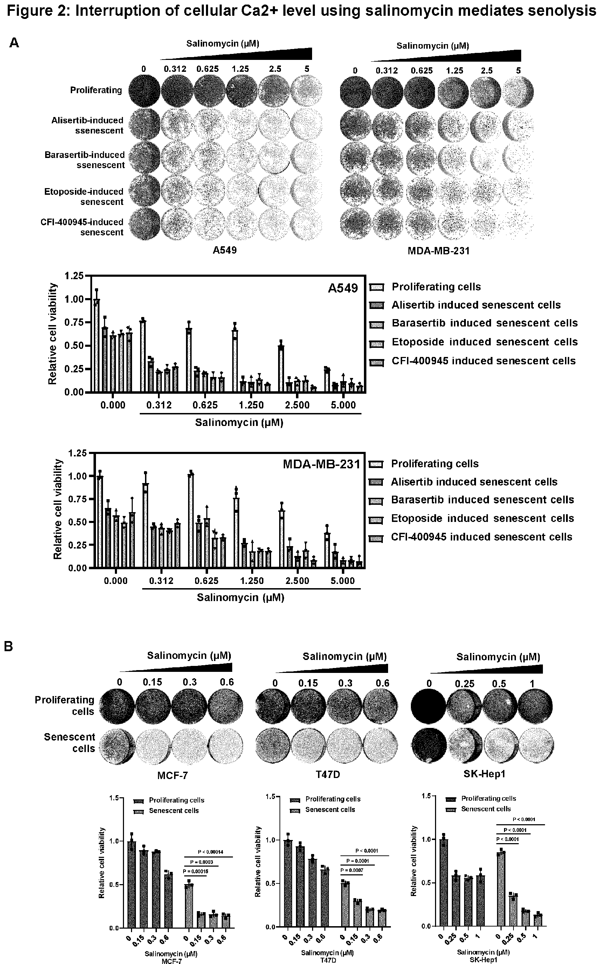

**Figure 2****: the calcium homeostasis disruptor salinomycin mediates senolysis**
   (A). Cell viability was assessed by colony formation assay in the A549 and MDA-MB-231 cells. The cells were firstly treated with independent senescence inducers, 0.5 µM alisertib, 1 µM barasertib, 2 µM etoposide or 100nM CFI-400945 for 7 days to induce senescence. Afterwards, the senescent cells were suspended from the drugs, re-seeded in a 12-well plate then switched to salinomycin for 5 days. Proliferating cells were taken as a control.
   (B). Cell viability was assessed by colony formation assay in the MCF-7, T47D and SK-Hep1 cells. The cells were firstly treated with 0.5 µM alisertib for 7 days to induce senescence. Afterwards, the senescent cells were suspended from the drugs, re-seeded in a 12-well plate then switched to salinomycin for 5 days. Proliferating cells were taken as a control.
**Figure 3****: Senescent cells are primed and sensitive to pyroptosis**
   (A). Cell morphology were imaged from the 7 days, 0.5 µM alisertib treatment-induced senescent cells. The cells were further treated with 1 µM salinomycin for 3 days. Proliferating cells were taken as a control.
   (B). Western blot analysis of the biomarker of extrinsic apoptosis, necroptosis, pyroptosis and hyper-autophage in alisertib-induced senescent and proliferating A549. Protein lysate were harvested after 24 hours 2 µM salinomycin treatment. HSP90 served as loading control(C). Cell viability was assessed by cell titer blue assay in the Senescent A549 cells were treated with salinomycin in combination with death pathway inhibitors. Rescue of cell death is indicated by the log10(IC50). Senescence was induced by 7 days 0.5 µM alisertib treatment. Afterwards, the senescent cells were re-seeded in a 384 wells plate and pre-treated with the inhibitors after 24hrs. Cells where then treated with salinomycin after 4hrs. PAO was used as a functional positive control. (D). Real-time PCR analysis cJun, NF-kB2, Nrf2 and Keap1 in A549 cells stably infected with three independent shRNA-SLC25A23 lentiviral vectors. mRNA was harvested after 3 days post-puromycin selection. GAPDH served as loading conII.
   (E). Western blot analysis of DR5 induction in cJun, NF-kB2, Nrf2 and Keap1 knocked-down senescent A549 cells treated with salinomycin (salino). Vinculin (VINC) served as the loading control.
   (F). Western blot analysis of DR5 induction in A549 cells treated with salinomycin (salino) and N-Acetyl Cysteine (NAC). Vinculin (VINC) served as the loading control.

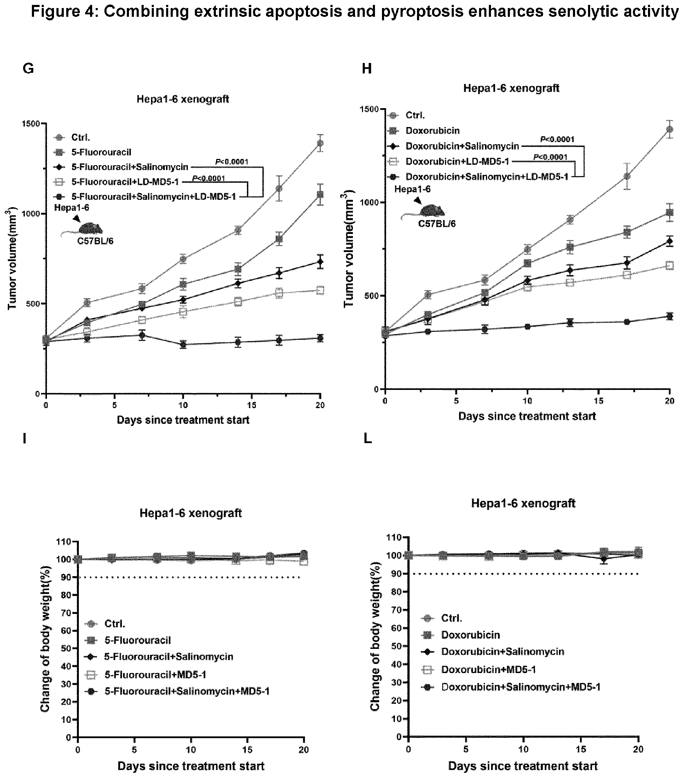

**Figure 4****: Combining DR5 agonist antibody and salinomycin enhances senolytic activity**
   (A). Cell viability was assessed by cell titer blue assay in the Senescent A549 cells were treated with salinomycin in combination DR5 activator conatumumab. A BLISS score above 10 is considered as synergistic. Senescence was induced by 7 days 0.5 µM alisertib treatment. Afterwards, the senescent cells were re-seeded in a 384 wells plate and co-treated with increasing concentrations of both salinomycin and conatumumab in a matrix gradient. PAO was used as a positive functional control.
   (B). Colony formation using low-dose of dimerized IgA-conatumumab (DR5 dIgA) and salinomycin on A549 cells made senescent by 1-week treatment of 0.5 µM alisertib. Proliferating cells were taken as a colol.
   (C). Tumor growth of A549 lung cancer cells in the flank immunodeficient nude mice. When tumors reached approximately 100 mm3, assigned to either vehicle control, 25 mg kg-1 alisertib, 1.5 mg kg-1 Salinomycin (Salino), 5 µg per dose conatumumab (Cona), the two-way combinations of 1.5 mg kg-1 Salinomycin (Salino) plus 5 µg per dose conatumumab and 25 mg kg-1 alisertib plus 5 µg per dose conatumumab, and then three-way combination of 25 mg kg-1 alisertib plus 1.5 mg kg-1 Salinomycin (Salino) plus 2.5 µg per dose conatumumab (n = 8 mice per group).
   (D). Body weight of in vivo experiment with A549 lung cancer cells into immunodeficient nude mice (n = 8 mice plgroup).
   (E). Tumor growth of Hepa1-6 liver cancer cells in syngeneic immunocompetent C57BL/6 mice. When tumors reached approximately 250 mm3, animals were assigned to either vehicle control, 25 mg kg-1 alisertib,1.5 mg kg-1 Salinomycin (Salino), 0.3 µg per dose MD5-1, the two-way combinations of 1.5 mg kg-1 Salinomycin (Salino) plus 0.3 µg per dose MD5-1 and 25 mg kg-1 alisertib plus 0.3 µg per dose MD5-1, and 1.5 mg kg-1 Salinomycin (Salino), and then three-way combination of 25 mg kg-1 alisertib plus 1.5 mg kg-1 Salinomycin (Salino) plus 0.3 µg per dose MD5-1 (n = 7 mice per group).
   (F). Body weight of in vivo experiment with Hepa1-6 liver cancer cells into immunocompetent C57BL/6 mice (n = 7 mice per group).
   (G). Tumor growth of Hepa1-6 liver cancer cells in syngeneic immunocompetent C57BL/6 mice. When tumors reached approximately 250 mm3, animals were assigned to either vehicle control, 40 mg kg-1 5-fluorouracil,1.5 mg kg-1 Salinomycin (Salino), 0.3 µg per dose MD5-1, the two-way combinations of 1.5 mg kg-1 Salinomycin (Salino) plus 0.3 µg per dose MD5-1 and 40 mg kg-1 5-fluorouracil plus 0.3 µg per dose MD5-1, and 1.5 mg kg-1 Salinomycin (Salino), and then three-way combination of 40 mg kg-1 5-fluorouracil plus 1.5 mg kg-1 Salinomycin (Salino) plus 0.3 µg per dose MD5-1 (n = 7 mice per group).
   (H). Tumor growth of Hepa1-6 liver cancer cells in syngeneic immunocompetent C57BL/6 mice. When tumors reached approximately 250 mm3, animals were assigned to either vehicle control, 3 mg kg-1 doxorubicin,1.5 mg kg-1 Salinomycin (Salino), 0.3 µg per dose MD5-1, the two-way combinations of 1.5 mg kg-1 Salinomycin (Salino) plus 0.3 µg per dose MD5-1 and 3 mg kg-1 doxorubicin plus 0.3 µg per dose MD5-1, and 1.5 mg kg-1 Salinomycin (Salino), and then three-way combination of 3 mg kg-1 doxorubicin plus 1.5 mg kg-1 Salinomycin (Salino) plus 0.3 µg per dose MD5-1 (n = 7 mice per group).
   (I). Body weight of in vivo experiment with Hepa1-6 liver cancer cells into immunocompetent C57BL/6 mice (n = 7 mice per group).
   (L). Body weight of in vivo experiment with Hepa1-6 liver cancer cells into immunocompetent C57BL/6 mice (n = 7 mice per group).

### Examples

### Cell lines

All cell lines were obtained from ATCC. Cell lines A549 were cultured in a RPMI-based medium supplemented with 10% FBS, 1% penicillin/streptomycin and 2 mM L-glutamine. Cell lines SK-Hep1, MB-MDA-231, T47D and MCF-7 were cultured in a DMEM-based medium supplemented with 10% FBS, 1% penicillin/streptomycin and 2 mM L-glutamine. Monoclonal A549-iCas9 were generated from A549, the cells infected by the edit-R Inducible Lentiviral Cas9 vector. All the Cell lines have been validated by STR profiling and regularly tested for Mycoplasma spp with PCR-based assay.

### Compounds and antibodies

Alisertib (#201931), Barasertib (#200420), Etoposide (#100330), CFI-400945 (#S7552) were purchased from Selleck Chemicals. Salinomycin (HY-15597) and nigericin (HY-127019) were purchased from Medchem Express. MCC950 (#522637), necrosulfonamide (#525389), 3MA (#406971), z-vad-fmk (#533011) and q-vd-0ph (#526845) were purchased from medkoo, biosciences inc. Lz-TRAIL (#LS-G3910) was purchased from LSBio. Dimerized IgA-conatumumab was purchased from UMAB. Anti-VINC (clone hVIN-1, #V9131) was purchased from Sigma-Aldrich. Anti-DR4 (polyclonal, #AB16955) and anti-DR5 (polyclonal, #AB16942) were purchased from MERCK. Anti-NRF2 (polyclonal, #ab137550) was purchased from Abcam. Anti- cleaved Gasdermin E (Asp270) (Clone E8G4U, #55879), Anti-Cleaved Gasdermin D (Asp275) (E7H9G, #36425), Anti-phospho-Rb (Ser780) (polyclonal, # 39033), Anti-P21 (polyclonal, #2947), Anti-Keap1 (monoclonal, #3073), Anti-p-mlkl (monoclonal, #91689), Anti-mlkl (monoclonal, #14993) were purchased from Cell Signaling Technology. Anti-HSP90 (polyclonal, #sc-7947), Anti-cJun (polyclonal, #sc-1694), Anti-NFKB p65 (polyclonal, #sc372) were purchased from Santa Cruz Biotechnology. Anti-Caspase 1 (polyclonal, # 22915-1-AP) was purchased from Proteintech.

### Plasmids and Primers

All the primers and individual shRNA vectors were collected from the TRC library. gRNA cloning vector pLentiGuide-puro (#52963), Human CRISPR Knockout Pooled Library (Brunello) (#73178)41 were purchased from addgene. The edit-R Inducible Lentiviral Cas9 vector (#NC1606271) was purchased from Dharmacon.

### iCas9-associated senolytic CRISPR screen

A549 were infected with a doxycycline-inducible Cas9 (iCas9) lentiviral vector to generate an inducible Cas9 cell model (A549-iCas9). Next, Brunello lentiviral whole genome-wide gRNA collection virus was introduced to the A549-iCas9 cells. These infected cells were firstly treated with 0.5 M alisertib for 7 days to drive them into senescence. Afterward, these cells were suspended from alisertib then switched to 1 µg/ml doxycycline treatment for 10 days. non-senescent cells were included as the control arm to filter out the straight lethal genes and also treated with doxycycline for. Changes in library representation after 10 days doxycycline treatment were determined by Illumina deep-sequencing. gRNAs prioritized for further analysis were selected by the fold depletion of abundance in in T2 and Tc samples compared with that in T1 and T0 samples, using methods as described previously (Evers et al., Nat Biotech, 2016).

### Protein lysate preparation and immunoblots

Cells were washed with PBS and lysed with RIPA buffer supplemented with protease inhibitor (cOmplete, Roche) and Phosphatase Inhibitor Cocktails II and III (Sigma). All lysates were freshly prepared and processed with Novex NuPAGE Gel Electrophoresis Systems (Invitrogen).

### Long-term cell proliferation assays

Cells were seeded into 6-well or 12-well plates and cultured both in the absence and presence of drugs as indicated. Within each cell line, cells cultured at different conditions were fixed with 4% paraformaldehyde (in PBS) at the same time. Afterwards, cells were stained with 0.1% crystal violet (in water).

### RNA isolation and qRT-PCR

Cells were harvested with TRIzol^{®} reagent (Invitrogen) following the manufacture's instruction. cDNA synthesis was performed using Maxima Universal First Strand cDNA Synthesis Kit (# K1661, Thermo scientific). qRT-PCR assays were performed using 7500 Fast Real-Time PCR System (Applied Biosystems). Relative mRNA levels of genes shown were normalized to the mRNA level of GAPDH (housekeeping gene). The primer sequences for assays using SYBR Green master mix (Roche) are as follows:
GAPDH Forward, AAGGTGAAGGTCGGAGTCAA;
GAPDH Reverse, AATGAAGGGGTCATTGATGG;
SLC25A23 Forward: AGGAATACCTGGCATTCCAG
SLC25A23 Reverse: CCAAGGAGAGCACCTTGAAA
cJUN forwards: TCCAAGTGCCGAAAAAGGAAG
cJUN reverse CGAGTTCTGAGCTTTCAAGGT
NFKB2 Forward: AGAGGCTTCCGATTTCGATATGG
NFKB2 Reverse GGATAGGTCTTTCGGCCCTTC
NRF2 Forward: TCCAGTCAGAAACCAGTGGAT
NRF2 Reverse GAATGTCTGCGCCAAAAGCTG
Keap1 Forward: CTGGAGGATCATACCAAGCAGG
Keap1 Reverse: GGATACCCTCAATGGACACCAC

### Cell line-derived xenografts

The maximal tumor size/burden was 2,000 mm3. All in vivo experiments did not exceed the maximal tumor size. Male 6-8-week-old BALB/c nude mice were used in the studies. A549 cells (1 × 107 cells per mouse) were injected subcutaneously into the flanks of nude mice. After tumor establishment, mice were randomly assigned to 5 d per week treatment (no drug at the weekend) and given vehicle, conatumumab (intraperitoneal injection), alisertib (oral gavage), salinomycin (oral gavage) or combinations. Hepa1-6 mouse liver cancer line cells (1 × 107 cells per mouse) were injected subcutaneously into the flanks of in syngeneic immunocompetent C57BL/6 mice (nine mice per group). After tumor establishment, mice were randomly assigned to 5 d per week treatment (no drug at the weekend) and given vehicle, alisertib, salinomycin or related combinations. In the treatment arms involved mouse DR5 agonist MD5-1, MD5-1 were intraperitoneal injected twice per week on every Monday and Thursday. Tumor volume based on caliper measurements was calculated by the modified ellipsoidal formula of tumor volume = ½ length × width2. Data are presented as mean ± s.e.m. Mice that showed therapy-induced adverse events in the early phases of treatment, precluding conclusions on the treatment efficacy, were excluded from longitudinal analyses. Exclusion criteria were predetermined before the experiments.

## Claims

1. A calcium homeostasis disruptor in combination with a selective Death Receptor 5 (DR5) agonist for use in a method of treating a patient suffering from a tumor.

2. The calcium homeostasis disruptor for use according to claim 1, wherein the disruptor interacts with the mitochondrial inner membrane protein SLC25A23.

3. The calcium homeostasis disruptor for use according to claim 1 and 2, wherein the disruptor is the ionophore antibiotic salinomycin.

4. The calcium homeostasis disruptor for use according to any one of claims 1 - 3, wherein the patient has been exposed to a treatment that induces senescence in tumor cells prior to the treatment according to any one of claims 1 - 3.

5. The calcium homeostasis disruptor for use according to claim 4, wherein the senescence inducer comprises at least one of palbociclib, alisertib, TAS-119, PF-06873600, CFI-400945, etoposide, doxorubicin, and barasertib, gamma-radiation.

6. The calcium homeostasis disruptor for use according to claim 4, wherein the inducer of senescence comprises at least one of chemotherapy, ionizing radiation, a CDK4/6 inhibitor, a polo-like kinase 4 (PLK4) inhibitor, a topoisomerase II inhibitor, an aurora kinase B inhibitor.

7. The calcium homeostasis disruptor for use according any one of claims 4-6, wherein the inducer of senescence, the calcium homeostasis disruptor and the selective Death Receptor 5 (DR5) agonist are provided sequentially to the patient.

8. The calcium homeostasis disruptor for use according any one of claims 1 - 7, wherein the tumor is a solid tumor such as lung cancer, breast cancer, colorectal cancer and/liver cancer.

9. The selective DR5 agonist for use according any one of claims 1 - 7, wherein the selective DR5 agonist is an antibody, preferably a human or humanized antibody, more preferably a human or humanized IgA or IgA-like antibody.

10. A pharmaceutical composition, comprising an inducer of senescence a calcium homeostasis disruptor and a selective Death Receptor 5 (DR5) agonist.

11. The pharmaceutical preparation according to claim 10, for use in a method of treating a patient suffering from a tumor.

12. A method of treating a patient having a tumor with a combination of an inducer of senescence, a calcium homeostasis disruptor and a selective DR5 agonist, comprising administering an inducer of senescence to said patient, followed by administering a calcium homeostasis disruptor followed by administering a selective DR5 agonist having an in vivo half-life of less than 20 days, optionally in combination with a BRD2 inhibitor.

13. The method of claim 12, wherein the calcium homeostasis disruptor is provided at least 24 hours following the inducer of senescence.

14. The method of claim 12, wherein the DR5 agonist is provided at least 48 hours following the inducer of senescence.

15. The method according to any one of claims 12-14, wherein the tumor is a solid tumor such as lung cancer, breast cancer, colorectal cancer and/or liver cancer.

16. A method of treating a patient having a pathology involving senescent cells with a calcium homeostasis disruptor followed by a selective DR5 agonist.
